# EUROPEAN PATENT SPECIFICATION

(11) **EP 3 818 968 B1**
(45) Date of publication and mention of the grant of the patent: **17.01.2024**
(21) Application number: 19207207.2
(22) Date of filing: 05.11.2019
(51) Int. Cl.: A61F 13/00

(54) **ADHESIVE MEDICAL ARTICLE HAVING FLUID MANAGEMENT PROPERTIES**
MEDIZINISCHER HAFTARTIKEL MIT FLÜSSIGKEITSMANAGEMENTEIGENSCHAFTEN
ARTICLE MÉDICAL ADHÉSIF AYANT DES PROPRIÉTÉS DE GESTION DE FLUIDE

(43) Date of publication of application: 12.05.2021
(73) Proprietor: 3M Innovative Properties Company, Saint Paul, MN 55133-3427 (US)
(72) Inventor: Ishiwatari, Hironobu, 41453 Neuss (DE); Apeldorn, Thomas, 41453 Neuss (DE); Peterson, Donald George, Saint Paul, Minnesota 55133-3427 (US); Popko, Daniel Thomas, Saint Paul, Minnesota 55133-3427 (US); Schuell, Christoph, 41453 Neuss (DE); Hannen, Thomas, 41453 Neuss (DE); Welke, Siegfried, 41453 Neuss (DE); Karpanen, Tarja, 41453 Neuss (DE); Grau, Uwe, 40721 Hilden (DE)
(74) Representative: Mathys & Squire

(56) References cited:
- EP-A1- 3 398 619
- WO-A1-97/07759
- FR-A1- 2 425 240
- US-A1- 2012 123 220

## Description

### Technical Field

The present disclosure relates generally to the field of medical articles, more specifically to the field of adhesive medical articles having fluid management properties. The present disclosure also relates to a method of manufacturing such adhesive medical articles.

### Background

Adhesive articles used in medical applications, such as wound dressings, are generally placed against the skin of a human body for a predetermined period of time specifically at a wound site. The medical adhesive articles are typically required to possess a certain set of properties such as e.g. good securement, gentleness to skin, moisture- and air-permeability and conformability.

When medical wound dressings are meant to be used during or after surgery operations, they are additionally required to possess excellent biological fluids (in particular wound exudates) control and/or transportation properties. Such proper fluid management is typically desired to maintain an aseptic environment and minimize infection risk at the wound or medically treated site, or to prevent biological fluids spillage. Adequate fluid management will also typically result into preserving better adhesive securement and improved comfort for the patient. In some other instances, the biological fluids, in particular blood, might need to be collected and recycled back to the patient.

Medical articles and systems known in the art for providing biological fluids control typically rely on four different types of technical solution: a) the use of absorbent pads directly over the wound or medically treated site; b) the use of suction tubes; c) the use of films or materials having a high moisture vapor transmission rate (MVTR) or d) the use of engineered films provided with micro-replicated channels. All these known solutions suffer from various limitations or deficiencies that significantly hamper the desired fluid management properties. The use of suction tubes, which may be employed to collect fluids from a wound site, typical create significant patient discomfort and can be source of infection. The use of high MVTR material and engineered films are typically insufficient to evacuate excess biological fluids from the so-called highly exuding wounds, such as intravenous (IV) sites or sites for gastric tubes. Films provided with micro-structured channels additional suffer from manufacturing complexity or production cost ineffectiveness. As for absorbent pads, they typically allow absorbing large volumes of fluid but are not fully satisfactory in terms of providing absorption speed sufficient to prevent fluid buildup. Also, absorbent pads are typically placed directly over the wound or medically treated site, which prevents visual observation of wound healing progression.

FR2425240 and WO 97/07759 disclose adhesive medical articles with a pattern of pressure sensitive adhesives.

Without contesting the technical advantages associated with the solutions known in the art, there is still a need for a medical adhesive article which overcomes the above-mentioned deficiencies. In particular, it is desirable to provide a medical adhesive article having excellent fluid management characteristics, whilst providing an optimum balance of securement and gentleness to skin.

### Summary

According to one aspect, the present invention relates to an adhesive medical article comprising a backing layer having a first major surface and a second major surface opposite to the first major surface, wherein the backing layer comprises a central portion and an outer circumferential portion, wherein the article further comprises a pressure sensitive adhesive pattern coating applied onto at least part of the first major surface of the backing layer in such a way as to form a plurality of adhesive coated and adhesive uncoated areas of the backing layer, wherein the adhesive uncoated areas comprise a central collection portion and at least one longitudinal extension radiating from the central collection portion towards the outer circumferential portion of the backing layer, wherein the pressure sensitive adhesive is selected from the group consisting of hydrophobic pressure sensitive adhesives.

According to another aspect, the present invention is directed to a method of manufacturing an adhesive medical article, comprising the steps of:
a) providing a backing layer having a first major surface and a second major surface opposite to the first major surface, and wherein the backing layer comprises a central portion and an outer circumferential portion; and
b) applying a pressure sensitive adhesive pattern coating onto at least part of the first major surface of the backing layer in such a way as to form a plurality of adhesive coated and adhesive uncoated areas of the backing layer, wherein the adhesive uncoated areas comprise a central collection portion and a plurality of longitudinal extensions radiating from the central collection portion towards the outer circumferential portion of the backing layer, wherein the pressure sensitive adhesive is selected from the group consisting of hydrophobic pressure sensitive adhesives.

### Brief Description of the Drawings

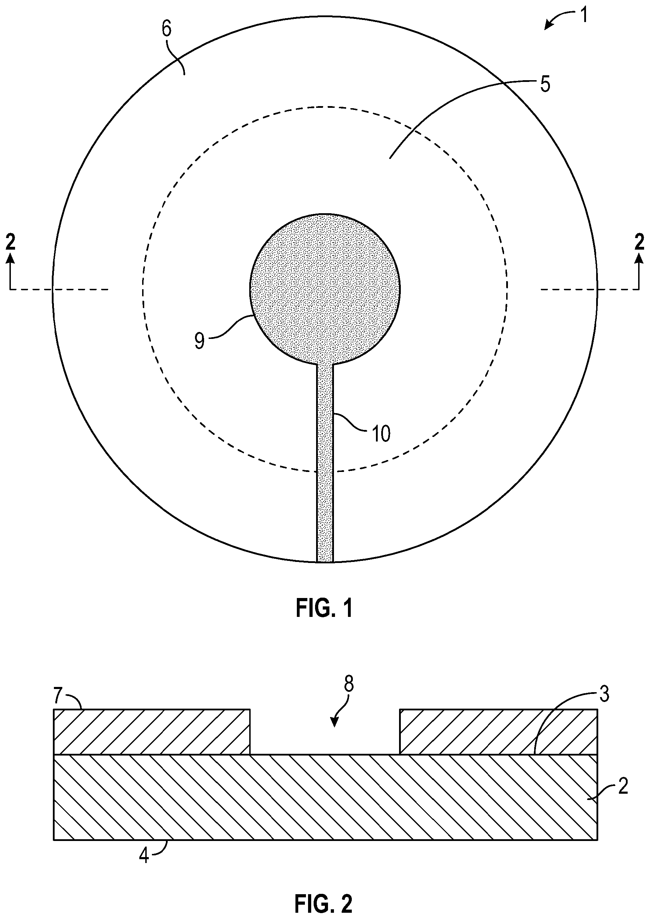

**FIG. 1** illustrates a top plan view of an adhesive medical article according to one aspect of the present disclosure, wherein the article comprises a central collection portion and one longitudinal extension having a linear shape.
**FIG. 2** is a cross-sectional view of the adhesive medical article represented in **FIG. 1** taken along the lines 2-2.

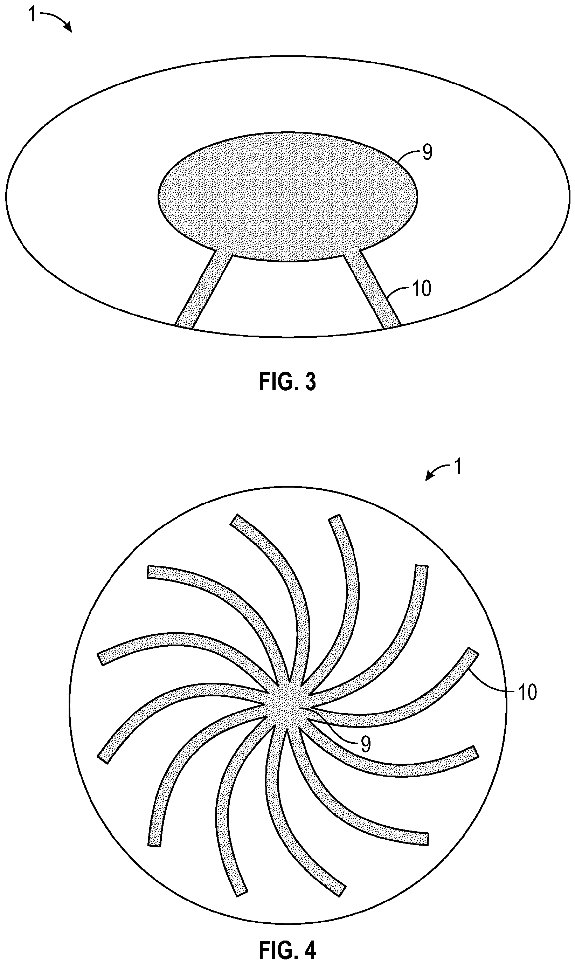

**FIG. 3** illustrates a top plan view of an adhesive medical article according to another aspect of the present disclosure, wherein the article comprises a central collection portion and two longitudinal extensions having a linear shape.
**FIG. 4** illustrates a top plan view of an adhesive medical article according to another aspect of the present disclosure, wherein the article comprises a central collection portion and a plurality of longitudinal extensions having a non-linear shape.

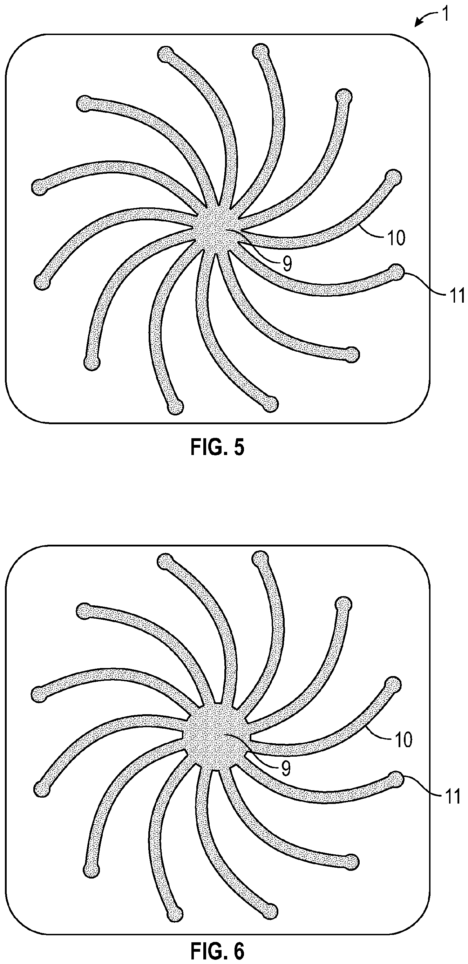

**FIG. 5** illustrates a top plan view of an adhesive medical article according to another aspect of the present disclosure, wherein the article comprises a central collection portion and a plurality of longitudinal extensions having a non-linear shape, and wherein each longitudinal extension comprises at its extremity a terminal portion.
**FIG. 6** illustrates a top plan view of an adhesive medical article according to another aspect of the present disclosure, wherein the article comprises a central collection portion and a plurality of longitudinal extensions having a non-linear shape, and wherein each longitudinal extension comprises at its extremity a terminal portion.

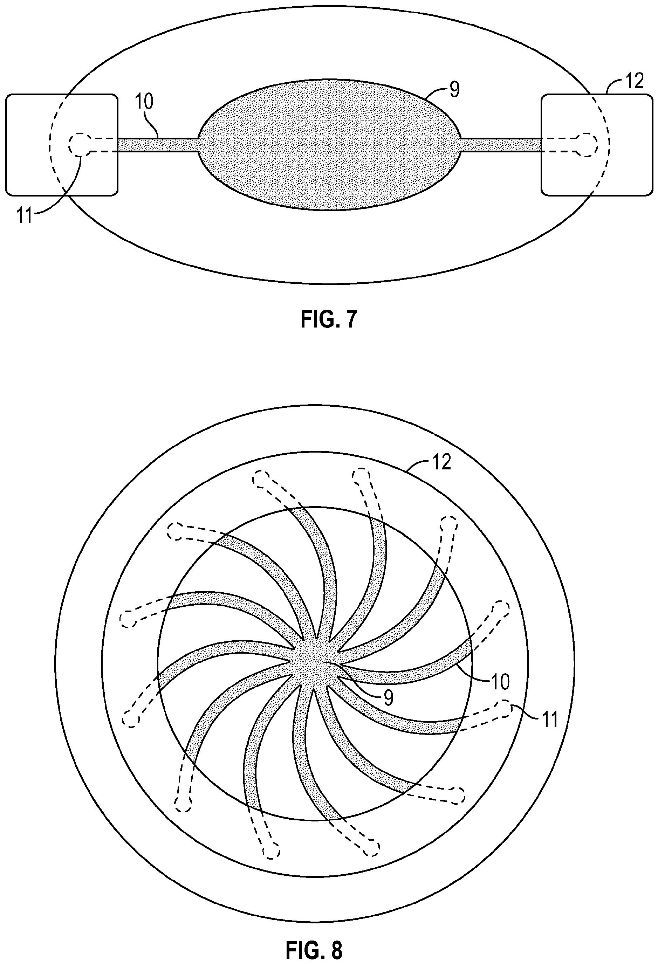

**FIG. 7** illustrates a top plan view of an adhesive medical article according to another aspect of the present disclosure, wherein the article comprises a central collection portion, two longitudinal extensions having a linear shape and two absorbent pads.
**FIG. 8** illustrates a top plan view of an adhesive medical article according to another aspect of the present disclosure, wherein the article comprises a central collection portion, a plurality of longitudinal extensions having a non-linear shape, and a rounded-shape single absorbent pad.

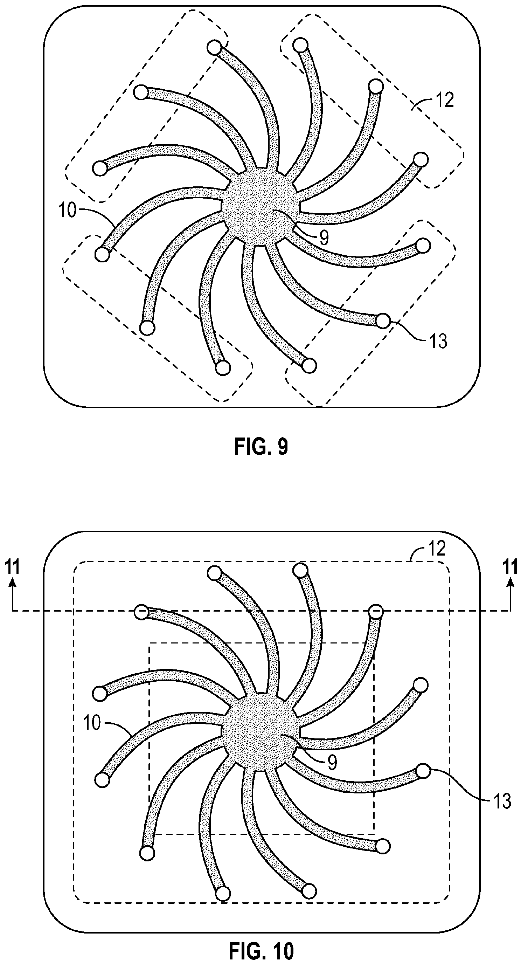

**FIG. 9** illustrates a top plan view of an adhesive medical article according to another aspect of the present disclosure, wherein the article comprises a central collection portion and a plurality of longitudinal extensions having a non-linear shape, wherein each longitudinal extension comprises at its extremity a terminal portion, wherein the article is further provided with a plurality of through hole perforations overlapping with a plurality of corresponding terminal portions and with four absorbing pads each connected to some terminal portions through the corresponding perforations.
**FIG. 10** illustrates a top plan view of the adhesive medical article according to another aspect of the present disclosure, wherein the article comprises a central collection portion and a plurality of longitudinal extensions having a non-linear shape, wherein each longitudinal extension comprises at its extremity a terminal portion, wherein the article is further provided with a plurality of through hole perforations overlapping with a plurality of corresponding terminal portions, and wherein the article comprises a single absorbent pad which is connected to all the terminal portions through all the corresponding perforations.

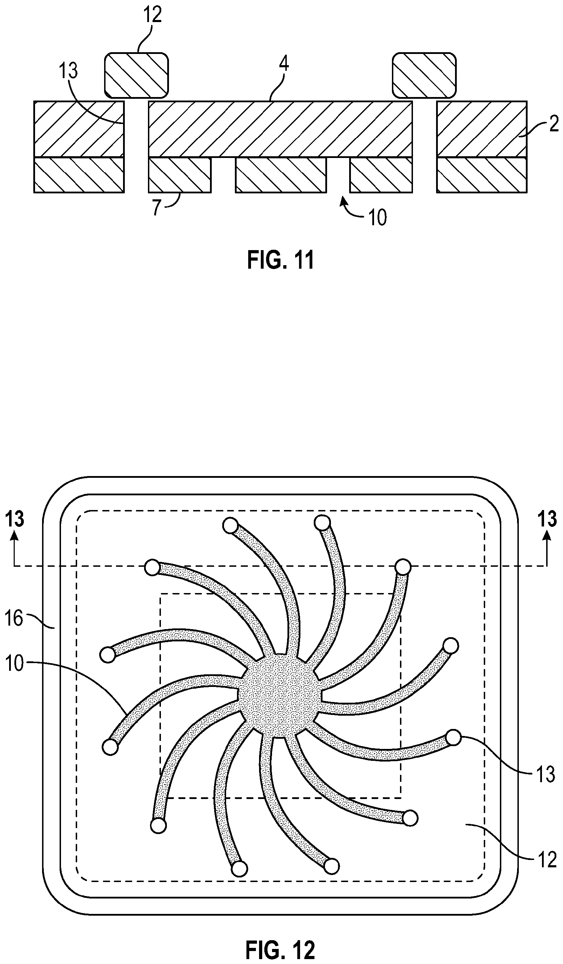

**FIG. 11** is a cross-sectional view of the adhesive medical article represented in **FIG. 10** taken along the lines 11-11.
**FIG. 12** is a top plan view of the adhesive medical article represented in **FIG. 10****,** which is further provided with an exemplary cover film.

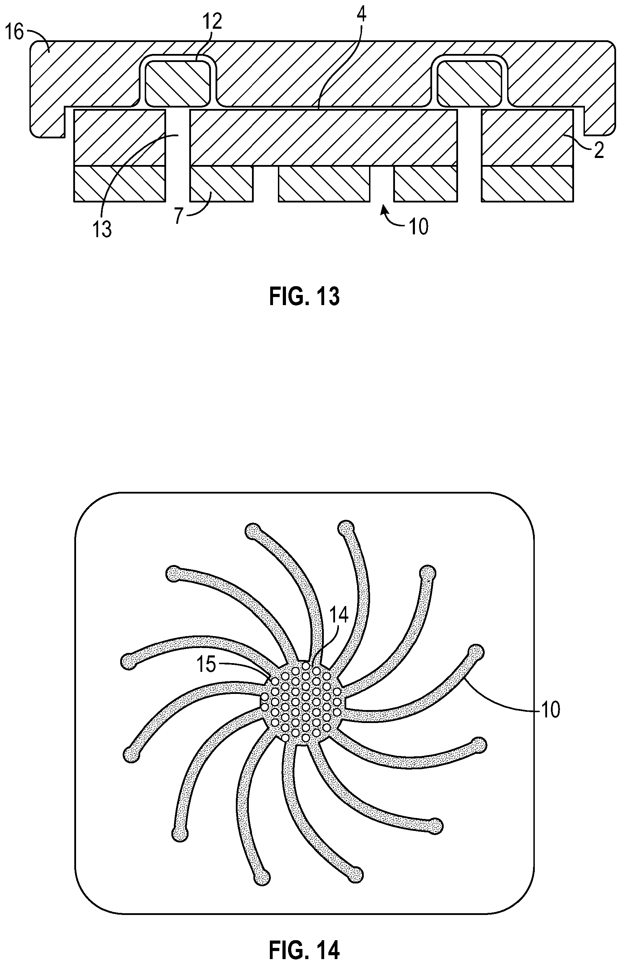

**FIG. 13** is a cross-sectional view of the adhesive medical article represented in **FIG. 12** taken along the lines 13-13.
**FIG. 14** illustrates a top plan view of an adhesive medical article according to another aspect of the present disclosure, wherein the article comprises a central collection portion and a plurality of longitudinal extensions having a non-linear shape, and wherein the article is further provided with a secondary pressure sensitive adhesive pattern coating applied onto at least part of the surface of the central collection portion.

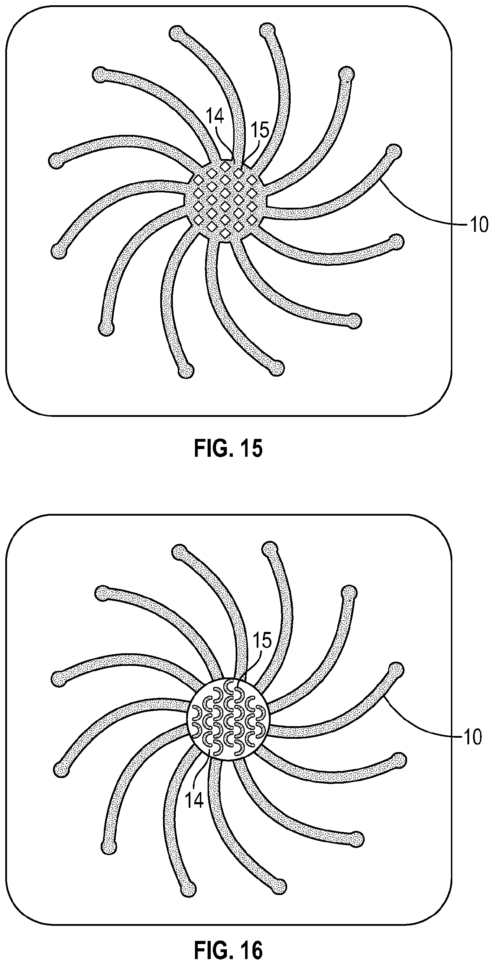

**FIG. 15** illustrates a top plan view of an adhesive medical article according to another aspect of the present disclosure, wherein the article comprises a central collection portion and a plurality of longitudinal extensions having a non-linear shape, and wherein the article is further provided with an alternative secondary pressure sensitive adhesive pattern coating applied onto at least part of the surface of the central collection portion.
**FIG. 16** illustrates a top plan view of an adhesive medical article according to another aspect of the present disclosure, wherein the article comprises a central collection portion and a plurality of longitudinal extensions having a non-linear shape, and wherein the article is further provided with an alternative secondary pressure sensitive adhesive pattern coating applied onto at least part of the surface of the central collection portion.

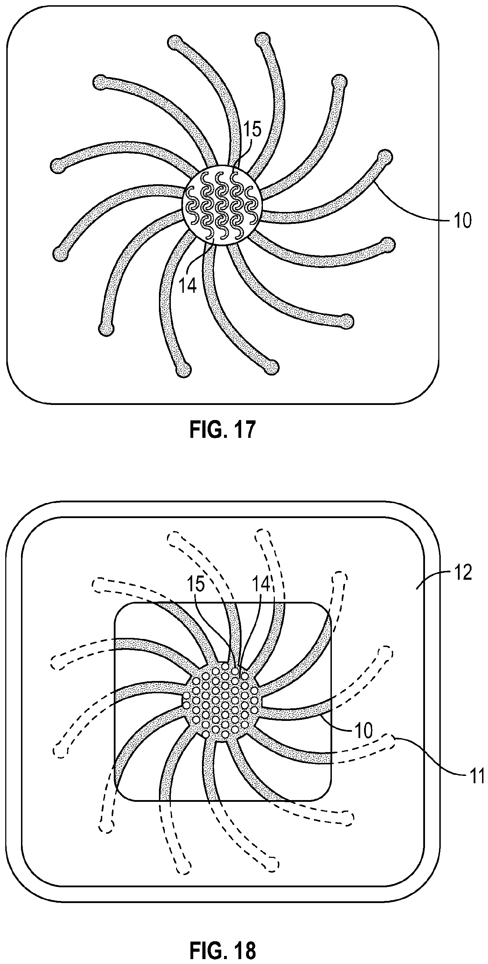

**FIG. 17** illustrates a top plan view of an adhesive medical article according to another aspect of the present disclosure, wherein the article comprises a central collection portion and a plurality of longitudinal extensions having a non-linear shape, and wherein the article is further provided with an alternative secondary pressure sensitive adhesive pattern coating applied onto at least part of the surface of the central collection portion.
**FIG. 18** is a top plan view of an adhesive medical article according to another aspect of the present disclosure, wherein the article comprises a central collection portion and a plurality of longitudinal extensions having a non-linear shape, wherein the article is further provided with a secondary pressure sensitive adhesive pattern coating applied onto at least part of the surface of the central collection portion, wherein each longitudinal extension comprises at its extremity a terminal portion, and wherein the article comprises a single absorbent pad which is connected to all the terminal portions.

### Detailed description

According to a first aspect, the present invention relates to an adhesive medical article comprising a backing layer having a first major surface and a second major surface opposite to the first major surface, wherein the backing layer comprises a central portion and an outer circumferential portion, wherein the article further comprises a pressure sensitive adhesive pattern coating applied onto at least part of the first major surface of the backing layer in such a way as to form a plurality of adhesive coated and adhesive uncoated areas of the backing layer, wherein the adhesive uncoated areas comprise a central collection portion and at least one longitudinal extension radiating from the central collection portion towards the outer circumferential portion of the backing layer.

In the context of the present disclosure, it has been surprisingly found that an adhesive medical article as described above is provided with excellent fluid management characteristics, as well as an optimum balance of adhesive securement and gentleness to skin. This is a particularly unexpected finding as these characteristics are known to be self-contradicting as such. In one advantageous aspect, the adhesive medical article of the present disclosure allows the medically treated site to remain visible not only at the time of application but also during the entire healing process. The adhesive medical article as described above is further characterized by reduced manufacturing complexity and associated production costs.

Without wishing to be bound by theory, it is believed that these excellent characteristics and performance attributes are due in particular to the presence of the specific pressure sensitive adhesive pattern coating applied onto at least part of the first major surface of the backing layer in such a way as to form a plurality of adhesive coated and adhesive uncoated areas of the backing layer, wherein the uncoated areas comprise a central collection portion and at least one longitudinal extension radiating from the central collection portion towards the outer circumferential portion of the backing layer. The improved visibility attribute is due, in particular, to the presence of the central collection portion which is part of the adhesive uncoated areas.

In the context of the present disclosure, the expression "central portion of the backing layer" is meant to designate the region of the backing layer which is situated between the intersection point (I) of all the axis of symmetry of the backing layer and less than half of the distance between the intersection point (I) and the extremity of the backing layer. The expression "outer circumferential portion of the backing layer" is meant to refer to the region of the backing layer which is situated between the extremity of the backing layer and half of the distance between the extremity of the backing layer and the intersection point (I).

The adhesive medical article of the present disclosure comprises a backing layer which is provided with uncoated areas comprising a central collection portion. The central collection portion for use herein is typically meant to collect the biological fluids exuding from the wound or medically-treated site. Shape and size of the central collection portions for use herein are not particularly limited. Suitable shapes and sizes of the central collection portions will be easily identified by those skilled in the art in the light of the present disclosure.

**FIG. 1** is a top plan view of a round-shaped adhesive medical article **1** according to one aspect of the present disclosure, wherein the article comprises a backing layer having a central portion **5** and an outer circumferential portion **6,** wherein the backing layer is provided on its first major surface with a pressure sensitive adhesive pattern coating comprising a plurality of adhesive coated and adhesive uncoated areas of the backing layer, wherein the adhesive uncoated areas comprise a central collection portion **9** and one longitudinal extension **10** having a linear shape and radiating from the central collection portion **9** towards the outer circumferential portion **6** of the backing layer.

**FIG. 2** is a cross-sectional view of the adhesive medical article represented in **FIG. 1** taken along the lines 2-2, and which depicts the backing layer **2** comprising a first major surface **3** and a second major surface **4,** and which is provided on its first major surface **3** with a pressure sensitive adhesive pattern coating comprising a plurality of adhesive coated areas **7** and adhesive uncoated areas **8** of the backing layer **4.**

In one exemplary aspect, the central collection portion for use herein has a shape selected from the group consisting of circle, oval, square, triangle, diamond, polygon, and any combinations thereof.

In another exemplary aspect, the central collection portion has a size (of the greatest dimension) in a range from 1 to 400 mm, from 1 to 350 mm, from 1 to 300 mm, from 1 to 250 mm, from 1 to 200 mm, from 1 to 150 mm, from 1 to 100 mm, from 1 to 80 mm, from 1 to 50 mm, from 1 to 30 mm, from 2 to 30 mm, from 2 to 25 mm, from 2 to 20 mm, from 3 to 20 mm, or even from 5 to 15 mm.

In one advantageous aspect, the central collection portion has a (substantial) circular shape having in particular a diameter in a range from 1 to 30 mm, from 2 to 30 mm, from 2 to 25 mm, from 2 to 20 mm, from 3 to 20 mm, or even from 5 to 15 mm.

According to one advantageous aspect of the disclosure, the adhesive medical article comprises a plurality of longitudinal extensions radiating from the central collection portion towards the outer circumferential portion of the backing layer. The presence of a plurality of longitudinal extensions radiating from the central collection portion towards the outer circumferential portion of the backing layer is believed to accelerate the biological fluid transport away from the wound or medically-treated site, in particular by increasing the volume of biological fluids being transported through the longitudinal extensions. The plurality of longitudinal extensions, when compared to a single longitudinal extension is also believed to advantageously affect the gentleness to skin attribute, while preserving adequate adhesive securement.

According to another advantageous aspect of the disclosure, the longitudinal extensions have a substantially radial configuration. In the context of the present disclosure, the expression "the longitudinal extensions have a substantially radial configuration" is meant to express that the longitudinal extensions radially extend toward the outer circumferential portion of the backing layer.

In a beneficial aspect of the disclosure, each longitudinal extension has a length-to-width ratio of at least 5, at least 10, at least 15, at least 20, at least 25, at least 30, at least 35, at least 40, at least 45, or even at least 50.

In another beneficial aspect of the disclosure, each longitudinal extension has a width of at least 0.5 mm, at least 1 mm, at least 1.5 mm, or even at least 2 mm.

In still another beneficial aspect of the disclosure, each longitudinal extension has a width no greater than 10 mm, no greater than 8 mm, no greater than 5 mm, no greater than 4 mm, no greater than 3 mm, or even no greater than 2 mm.

In still another beneficial aspect of the disclosure, each longitudinal extension has a width in a range from 0.5 to 10 mm, from 0.5 to 8 mm, from 0.5 to 5 mm, from 0.5 to 4 mm, from 1 to 4 mm, from 1 to 4 mm, from 1.5 to 4 mm, from 1.5 to 3.5 mm, from 1.5 to 3 mm, or even from 1.5 to 2.5 mm.

In yet another beneficial aspect of the disclosure, each longitudinal extension has a length of at least 10 mm, at least 20 mm, at least 30 mm, at least 35 mm, at least 40 mm, at least 40 mm, at least 50 mm, at least 55 mm, or even at least 60 mm.

In yet another advantageous aspect of the disclosure, each longitudinal extension has a length no greater than 200 mm, no greater than 180 mm, no greater than 160 mm, no greater than 150 mm, no greater than 140 mm, no greater than 120 mm, no greater than 100 mm, no greater than 90 mm, no greater than 80 mm, no greater than 70 mm, or even no greater than 65 mm.

In yet another advantageous aspect of the disclosure, each longitudinal extension has a length in a range from 10 to 200 mm, from 10 to 180 mm, from 10 to 150 mm, from 10 to 120 mm, from 10 to 100 mm, from 10 to 90 mm, from 20 to 90 mm, from 20 to 80 mm, from 30 to 80 mm, from 30 to 75 mm, from 35 to 75 mm, from 35 to 70 mm, from 40 to 70 mm, from 45 to 70 mm, from 45 to 65 mm, from 50 to 65 mm, or even from 50 to 60 mm.

According to a beneficial aspect of the disclosure, each longitudinal extension has a continuous and substantially non-linear shape.

In a particularly advantageous aspect, the longitudinal extension for use herein has a curved shape, in particular the shape of a curved strip. Longitudinal extensions having a curved shape are believed to beneficially impact the speed at which the biological fluid is being transported away from the wound or medically-treated site, in particular through improved guiding effect facilitated by the curved contours of the longitudinal extensions.

In an alternative aspect, the longitudinal extensions for use herein have a continuous and substantially linear shape, in particular the shape of a linear strip.

**FIG. 3** illustrates a top plan view of an oval-shaped adhesive medical article **1** according to another aspect of the present disclosure, wherein the article comprises a central collection portion **9** and two longitudinal extensions **10** having a linear shape.

**FIG. 4** illustrates a top plan view of a round-shaped adhesive medical article **1** according to another aspect of the present disclosure, wherein the article comprises a central collection portion **9** and a plurality of longitudinal extensions **10** having a non-linear shape.

According to a typical execution, each longitudinal extension for use in the adhesive medical article takes the form of a channel or a pathway in the adhesive coated area of the backing layer. The term channel or pathway suitably characterize the technical function of the longitudinal extensions which are meant to facilitate and beneficially impact the fluid management characteristics of the adhesive medical article.

In a beneficial aspect of the disclosure, the depth of each longitudinal extension for use herein is a range from 10 to 1000 micrometers, from 10 to 500 micrometers, from 10 to 400 micrometers, from 10 to 300 micrometers, from 10 to 200 micrometers, from 30 to 160 micrometers, from 40 to 160 micrometers, from 40 to 150 micrometers, from 50 to 150 micrometers, from 50 to 140 micrometers, from 60 to 140 micrometers, from 60 to 130 micrometers, from 70 to 130 micrometers, from 70 to 120 micrometers, from 80 to 120 micrometers, from 80 to 110 micrometers, from 90 to 110 micrometers, or even from 95 to 105 micrometers.

According to another advantageous aspect, the thickness of the pressure sensitive adhesive pattern coating applied onto at least part of the first major surface of the backing layer is in a range from 10 to 1000 micrometers, from 10 to 500 micrometers, from 10 to 400 micrometers, from 10 to 300 micrometers, from 10 to 200 micrometers, from 30 to 160 micrometers, from 40 to 160 micrometers, from 40 to 150 micrometers, from 50 to 150 micrometers, from 50 to 140 micrometers, from 60 to 140 micrometers, from 60 to 130 micrometers, from 70 to 130 micrometers, from 70 to 120 micrometers, from 80 to 120 micrometers, from 80 to 110 micrometers, from 90 to 110 micrometers, or even from 95 to 105 micrometers.

As will be easily apparent to those skilled in the art, the thickness of the pressure sensitive adhesive pattern coating applied onto the backing layer is directly correlated with the depth of the longitudinal extension(s).

In an exemplary aspect of the present disclosure, the average coat weight of the adhesive pattern coating applied onto at least part of the first major surface of the backing layer is in a range from 10 to 1000 g/m², from 10 to 500 g/m², from 10 to 400 g/m², from 10 to 300 g/m², from 10 to 200 g/m², 30 to 160 g/m², from 40 to 160 g/m², from 40 to 150 g/m², from 50 to 150 g/m², from 50 to 140 g/m², from 60 to 140 g/m², from 60 to 130 g/m², from 70 to 130 g/m², from 70 to 120 g/m², from 80 to 120 g/m², from 80 to 110 g/m², from 90 to 110 g/m², or even from 95 to 105 g/m².

The adhesive pattern coating for use herein may be applied onto at least part of the first major surface of the backing layer by any means known to those skilled in the art. Suitable means for forming the adhesive pattern coating for use herein include, but are not limited to, coating techniques, melt blowing techniques, and printing techniques.

Suitable coating techniques include, but are not limited to, roller coating, knife coating, dip-coating, and spray coating. Beneficially, the adhesive pattern coating for use herein may be applied from a hotmelt pressure sensitive adhesive material.

Suitable printing techniques for applying the adhesive pattern coating include gravure printing, block printing, intaglio printing, lithographic printing, rotary screen printing, flat screen printing, flexographic printing, and any combinations thereof.

According to one advantageous aspect of the disclosure, the adhesive pattern coating for use herein is applied onto at least part of the first major surface of the backing layer by screen printing in particular from a hotmelt adhesive material.

In one typical aspect of the disclosure, the adhesive pattern coating for use in the adhesive medical article forms a discontinuous pattern wherein the adhesive uncoated areas are interconnected. This interconnection between the adhesive uncoated areas of the backing layer is meant to facilitate and beneficially impact the fluid management characteristics of the adhesive medical article.

In another typical aspect of the adhesive medical article, the adhesive uncoated area(s) comprising the central collection portion and the plurality of longitudinal extensions are substantially fully surrounded by the adhesive coated area(s).

In still another typical aspect, the first major surface of the backing layer is substantially free of adhesive uncoated area(s) not comprising the central collection portion and the plurality of longitudinal extensions.

According to one advantageous aspect of the adhesive medical article, the total adhesive uncoated area comprising the central collection portion and the plurality of longitudinal extensions is no greater than 50%, no greater than 45%, no greater than 40%, no greater than 35%, no greater than 30%, no greater than 25%, no greater than 20%, no greater than 15%, no greater than 10%, or even no greater than 5%, of the total surface of the backing layer.

According to another advantageous aspect, the total adhesive uncoated area comprising the central collection portion and the plurality of longitudinal extensions is in a range from 2 to 50%, from 2 to 45%, from 5 to 45%, from 5 to 40%, from 5 to 35%, from 10 to 35%, from 10 to 30%, from 10 to 25 %, or even from 10 to 20%, of the total surface of the backing layer.

According to still another advantageous aspect, the total adhesive coated area is greater than 50%, greater than 55%, greater than 60%, greater than 65%, greater than 70%, greater than 75%, greater than 80%, greater than 85%, greater than 90%, or even greater than 95%, of the total surface of the backing layer.

According to yet another aspect of the disclosure, the total adhesive coated area is in a range from 50 to 98%, from 55 to 95%, from 55 to 90%, from 60 to 90%, from 65 to 90%, from 65 to 85%, from 70 to 85%, or even from 75 to 85%, of the total surface of the backing layer.

The advantageous distribution of adhesive coated area and adhesive uncoated area as detailed above, is believed to beneficially impact not only the fluid management performance of the adhesive medical article but also the reaching of an optimum balance of adhesive securement and gentleness to skin.

Pressure sensitive adhesive material for use herein are not particularly limited. Suitable pressure sensitive adhesives for use herein will be easily identified by those skilled in the art in the light of the present disclosure.

Exemplary pressure sensitive adhesives for use herein comprise a material selected from the group consisting of silicones, rubbers, block copolymers, acrylates, urethane, hydrogels, hydrocolloids, and any combinations or mixtures thereof.

According to the invention, the pressure sensitive adhesive for use herein is selected from the group consisting of hydrophobic pressure sensitive adhesives. As will be apparent to those skilled in the art, the expression "hydrophobic pressure sensitive adhesives" is meant to designate those pressure sensitive adhesives having no or low affinity with water, i.e. the ability to repel water. The use of hydrophobic pressure sensitive adhesives is believed to facilitate and beneficially impact the fluid management characteristics of the adhesive medical article, by specifically repelling (through electrostatic interactions) and guiding the aqueous biological fluid transport away from the wound or medically-treated site and through the longitudinal extensions.

In one advantageous aspect, the pressure sensitive adhesive for use herein is selected from the group consisting of silicone-based pressure sensitive adhesives, rubber-based pressure sensitive adhesives, acrylic-based pressure sensitive adhesives, and any combinations or mixtures thereof.

According to one particularly preferred aspect, the pressure sensitive adhesive for use herein is selected from the group consisting of silicone-based pressure sensitive adhesives, wherein the silicone base material is selected from the group of crosslinked polysiloxanes, in particular crosslinked poly diorganosiloxanes, more in particular poly dimethylsiloxanes. Silicone-based pressure sensitive adhesives are particularly advantageous in that they provide good adhesion to human skin with gentle removal force, which makes them particularly suitable for gentle to skin adhesive applications. Moreover, they have the ability to be repositioned and are provided with very advantageous transparency and hydrophobicity characteristics. Examples of silicone-based pressure sensitive adhesives for use herein and methods of manufacturing thereof are described e.g. in WO 2010/056544 (Determan et al.).

According to another advantageous aspect of the disclosure, the pressure sensitive adhesive for use herein is selected from the group consisting of rubber-based pressure sensitive adhesives, in particular those derived from styrene-isoprene-styrene (SIS) rubber, styrene-butadiene-styrene (SBS) rubber, styrene-butadiene (SB) rubber, nitrile butyl rubber (NBR), polyisobutylene (PIB) rubber, polyisoprene rubber, polybutadiene rubber, acrylic rubber, silicone rubber, urethane rubber, butyl rubber, ethylene-propylene rubber, fluoro rubber, polychloroprene rubber, halobutyl rubber, butadiene-acrylonitrile rubber, any combinations or mixtures thereof.

According to still another advantageous aspect, the pressure sensitive adhesive for use in the present disclosure is selected from the group consisting of acrylic-based pressure sensitive adhesives, in particular those derived from acrylic copolymers, more in particular isooctyl acrylate:acrylamide copolymer, isooctyl acrylate:ethyleneoxide acrylate: acrylic acid terpolymer, and any mixtures thereof.

According to yet another advantageous aspect, the pressure sensitive adhesive for use in the adhesive medical article comprises a blend of silicone-based pressure sensitive adhesives and acrylic-based pressure sensitive adhesives.

In another advantageous execution, the pressure sensitive adhesive is selected from the group consisting of urethane-based pressure sensitive adhesives, in particular those derived from urethane (co)polymers.

Backing layers for use herein are not particularly limited. Any backing layer commonly known in the art may be used in the context of the present disclosure. Suitable backing layers for use herein will be easily identified by those skilled in the art in the light of the present disclosure. Exemplary backing layers for use herein comprise a material selected from the group consisting of polymeric films, nonwoven fibrous webs, woven fibrous webs, knits, porous films, and any combinations thereof.

In one advantageous aspect of the disclosure, the backing layer for use herein comprises a material selected from the group consisting of polymeric films, in particular those derived from elastomeric polyurethane, polyester, polyether block amide, and any combinations or mixtures thereof.

Suitable backing materials are preferably chosen to have advantageous transparency or translucency characteristics, as well as high moisture vapor permeability and good conformability to anatomical surfaces. Examples of backing layers for use herein are described e.g. in US-A1-2001/0027285 (Heinecke et al.).

In a typical aspect of the adhesive medical article, the central collection portion is positioned in the central portion of the backing layer, and the central collection portion fully overlaps with the central portion of the backing layer.

According to a typical aspect of the adhesive medical article, each longitudinal extension for use herein comprises at its extremity a terminal portion which is positioned in the outer circumferential portion of the backing layer.

**FIG. 5** illustrates a top plan view of a square-shaped adhesive medical article **1** according to another aspect of the present disclosure, wherein the article comprises a central collection portion **9** and a plurality of longitudinal extensions **10** having a non-linear shape and wherein each longitudinal extension **10** comprises at its extremity a terminal portion **11.**

**FIG. 6** illustrates a top plan view of a square-shaped adhesive medical article **1** according to another aspect of the present disclosure, wherein the article comprises a larger central collection portion **9** and a plurality of longitudinal extensions **10** having a non-linear shape and provided with terminal portions **11.**

In one advantageous aspect of the disclosure, the terminal portions for use herein have a shape selected from the group consisting of circle, oval, square, triangle, diamond, polygon, and any combinations thereof.

In another advantageous aspect of the disclosure, each terminal portion has a size in a range from 0.5 to 5 mm, from 0.5 to 4 mm, from 1 to 4 mm, from 1 to 4 mm, from 1.5 to 4 mm, from 1.5 to 3.5 mm, from 1.5 to 3 mm, or even from 2 to 3 mm.

According to still another advantageous aspect, each terminal portion for use herein has a circular shape having a diameter in a range from 0.5 to 5 mm, from 0.5 to 4 mm, from 1 to 4 mm, from 1 to 4 mm, from 1.5 to 4 mm, from 1.5 to 3.5 mm, from 1.5 to 3 mm, or even from 2 to 3 mm.

In one beneficial aspect, the adhesive medical article of the present disclosure further comprises at least one absorbent pad connected to at least one terminal portion. In the context of the present disclosure, the expression "the absorbent pad is connected to a terminal portion" is meant to express that the absorbent pad is in fluid connection with the corresponding terminal portion, i.e. the absorbent pad is enabled to directly or indirectly absorb the biological fluid present in the corresponding terminal portion.

Absorbent pads for use herein are not particularly limited. Any absorbent pads commonly known in the art may be used in the context of the present disclosure. Suitable absorbent pads for use herein will be easily identified by those skilled in the art in the light of the present disclosure. Exemplary absorbent pads for use herein are described e.g. in US-A1-2001/0027285 (Heinecke et al.).

According to one more beneficial aspect of the adhesive medical article, all the longitudinal extensions for use herein comprise at their extremity a terminal portion which is positioned in the outer circumferential portion of the backing layer, and the article comprises a plurality of absorbent pads each connected to a corresponding terminal portion.

**FIG. 7** illustrates a top plan view of an oval-shaped adhesive medical article **1** according to another aspect of the present disclosure, wherein the article comprises a central collection portion **9,** two longitudinal extensions **10** having a linear shape and comprising at their extremity a terminal portion **11,** and wherein the article further comprises two absorbent pads **12** each connected to a corresponding terminal portion **11.**

According to another more beneficial aspect of the adhesive medical article, all the longitudinal extensions for use herein comprise at their extremity a terminal portion which is positioned in the outer circumferential portion of the backing layer, and wherein a single absorbent pad is connected to all the terminal portions.

Such a beneficial execution is described in **FIG. 8****,** which illustrates a top plan view of a round-shaped adhesive medical article **1** according to another aspect of the present disclosure, wherein the article **1** comprises a central collection portion **9,** a plurality of longitudinal extensions **10** having a non-linear shape and comprising at their extremity a terminal portion **11,** and wherein the article further comprises a rounded-shape single absorbent pad **12** connected to all the terminal portions **11.**

The presence of absorbent pad(s) configured such as to be in connection with their corresponding terminal portion(s) is believed to facilitate and beneficially impact the fluid management characteristics of the adhesive medical article, by specifically attracting (through capillarity and absorption/adsorption effects) the biological fluids away from the wound or medically-treated site and through the longitudinal extensions, and by efficiently collecting and scavenging the biological fluid exuding from the medically-treated site. The additional absorbent pad(s) are further believed to allow increased volume of biological fluids to be transported away from the wound or medically-treated site, while permitting the central collection portion to remain substantially free to collect additional fluids and maintaining an aseptic environment and minimizing infection risk at the wound or medically treated site.

Many different configurations may be envisaged for having the absorbent pad(s) suitably connected to their corresponding terminal portion(s).

In one advantageous aspect, the absorbent pad(s) are adhesively connected to the corresponding terminal portion(s), which means that the absorbent element (e.g. absorbing nonwoven material) of the absorbent pad(s) is placed directly over the surface of the corresponding terminal portion(s) while the carrier portion of the absorbent pad(s) which surrounds the absorbent element is adhesively secured to the surface surrounding the terminal portion(s), in particular via a suitable pressure sensitive adhesive.

In another advantageous aspect of the adhesive medical article, the absorbent pad(s) are releasably connected to the corresponding terminal portion(s), which means that the absorbent pad(s) may be conveniently and relatively easily detached or separated from the corresponding terminal portion(s). This specific configuration may be executed by using suitable pressure sensitive adhesives.

According to still another advantageous aspect, the absorbent pad(s) may be suitably positioned on the first major surface of the backing layer. This specific execution is particularly advantageous in that it provides better comfort to the patient due to the cushioning effect provided by the absorbent pad(s) and provides for an adhesive medical article having a relatively neat external surface.

Suitable absorbent pads for use in the context of the present disclosure may be formed by any absorbent material commonly known in the art. In one advantageous aspect, the absorbent pad for use herein comprises a material selected from the group consisting of woven, nonwoven cotton, rayon, synthetic or natural hydrophilic materials, hydrogels, hydrocolloid absorbent materials, foams, synthetic swelling polymers, synthetic or natural nonwoven materials, and any combinations or mixtures thereof.

In one particular aspect of the disclosure, the absorbent pad for use herein may comprise a hydrocolloid composition. The hydrocolloid absorbent may comprise, in particular, a natural hydrocolloid, such as pectin, gelatin, or carboxymethylcellulose (CMC) (Aqualon Corp., Wilmington, Del.), a semi-synthetic hydrocolloid, such as cross-linked carboxymethylcellulose (X4ink CMC) (e.g. Ac-Di-Sol; FMC Corp., Philadelphia, Pa.), a synthetic hydrocolloid, such as cross-linked polyacrylic acid (PAA) (e.g., CARBOPOL(TM) No. 974P; B.F. Goodrich, Brecksville, Ohio), or a combination thereof.

In another particular aspect, especially when the adhesive medical article is used as a drug delivery dressing, the absorbent may advantageously comprise any useful substance. Suitable substances for use with the absorbent pad may include, but are not limited to, antimicrobial agents, drugs for transdermal drug delivery, chemical indicators to monitor hormones or other substances in a patient, and any combinations thereof. Other alternative substances may also be used depending on the targeted application or therapeutic benefits.

According to the present disclosure, the longitudinal extension(s) for use in the adhesive medical article have the ability to drive an aqueous liquid from the central collection portion towards the outer circumferential portion of the backing layer, in particular towards the terminal portion of the longitudinal portion, and ultimately towards the optional absorbent pad(s). According to a typical aspect, the biological fluid or aqueous liquid referred to in the present disclosure is selected from human body fluids, in particular wound exudates.

According to an alternatively advantageous execution, the backing layer of the adhesive medical article may be provided with at least one through hole perforation overlapping with the at least one terminal portion, wherein the at least one absorbent pad is positioned on the second major surface of the backing layer and is connected to the at least one terminal portion through the at least one perforation.

In a more advantageous aspect, the backing layer of the adhesive medical article is provided with a plurality of through hole perforations overlapping with a plurality of corresponding terminal portions, wherein the article comprises a plurality of absorbent pads each connected to a corresponding terminal portion through the corresponding perforation.

**FIG. 9** illustrates a top plan view of a square-shaped adhesive medical article **1** according to another aspect of the present disclosure, wherein the article **1** comprises a central collection portion **9** and a plurality of longitudinal extensions **10** having a non-linear shape, wherein each longitudinal extension **10** comprises at its extremity a terminal portion, wherein the article **1** is further provided with a plurality of through hole perforations **13** overlapping with a plurality of corresponding terminal portions and with four rectangular-shaped absorbing pads **12,** wherein the absorbent pads **12** are positioned on the second major surface of the backing layer and are each connected to some terminal portions through the corresponding perforations **13.**

In another more advantageous aspect of the present disclosure, the backing layer is provided with a plurality of through hole perforations overlapping with a plurality of corresponding terminal portions, wherein the article comprises a single absorbent pad which is connected to all the terminal portions through all the corresponding perforations.

**FIG. 10** illustrates a top plan view of a square-shaped adhesive medical article **1** according to another aspect of the present disclosure, wherein the article **1** comprises a central collection portion **9** and a plurality of longitudinal extensions **10** having a non-linear shape, wherein each longitudinal extension **10** comprises at its extremity a terminal portion, wherein the article **1** is further provided with a plurality of through hole perforations **13** overlapping with a plurality of corresponding terminal portions, and wherein the article **1** comprises a single absorbent pad **12** positioned on the second major surface of the backing layer and connected to all the terminal portions through all the corresponding perforations **13.**

**FIG. 11** is a cross-sectional view of the adhesive medical article **1** represented in **FIG. 10** taken along the lines 11-11.

In still another more advantageous aspect of the present disclosure, the absorbent pad(s) for use herein are releasably connected to the corresponding terminal portion(s), in particular adhesively connected to the corresponding terminal portion(s).

According to the advantageous configuration according to which the absorbent pad(s) are connected to the corresponding terminal portion through the corresponding perforation(s), the absorbent pad(s) may be advantageously positioned on the second major surface of the backing layer, i.e. on the surface of the backing layer opposite to the first major surface carrying the pressure sensitive adhesive pattern coating. This particular configuration may be advantageously selected with the use of absorbent pad(s) releasably and/or adhesively connected to the corresponding terminal portion(s). This specific execution is particularly advantageous in that it allows the patient or the medical operator to conveniently remove and replace the absorbent pad(s) as they become saturated with biological fluids while significantly minimizing the discomfort or pain caused by the replacement operation. This is in particular due to the easy accessibility of the absorbent pad(s) which are releasably and/or adhesively connected to the corresponding terminal portion(s) through the corresponding perforation(s).

Suitable through hole perforations for use herein are not particularly limited as long as they overlap with the corresponding terminal portions and are able to have them connected to the corresponding absorbent pad(s). Suitable through hole perforations for use herein will be easily identified by those skilled in the art in the light of the present disclosure.

In one advantageous aspect of the disclosure, the through hole perforations for use herein have a shape selected from the group consisting of circle, oval, square, triangle, diamond, polygon, and any combinations thereof.

In another advantageous aspect of the disclosure, each through hole perforation has a in a range from 0.5 to 6 mm, from 0.5 to 5 mm, from 1 to 5 mm, from 2 to 5 mm, from 3 to 5 mm, or even from 3.5 to 4.5 mm.

According to still another advantageous aspect, each through hole perforation for use herein has a circular shape having a diameter in a range from 0.5 to 6 mm, from 0.5 to 5 mm, from 1 to 5 mm, from 2 to 5 mm, from 3 to 5 mm, or even from 3.5 to 4.5 mm.

In a beneficial aspect, the adhesive medical article of the present disclosure is further provided with a secondary pressure sensitive adhesive pattern coating applied onto at least part of the surface of the central collection portion in such a way as to form a plurality of secondary adhesive coated and adhesive uncoated areas of the central collection portion.

In the context of the present disclosure, it has been surprisingly found that the presence of a secondary pressure sensitive adhesive pattern coating as defined above is able to beneficially impact the overall fluid management characteristics of the adhesive medical article, in particular by modulating the volume and speed of the biological fluid transport away from the wound or medically-treated site. The secondary pressure sensitive adhesive pattern coating as defined above is also believed to advantageously affect the fine and delicate balance between the gentleness to skin attribute and the adhesive securement performance of the adhesive medical article.

The secondary pressure sensitive adhesive pattern coating may share a lot of similarities with the primary pressure sensitive adhesive pattern coating as defined above, in particular in terms of the shape of the coated and adhesive uncoated areas, the overall adhesive pattern, the depth of the adhesive coated areas, the average coat weight and the chemical composition of the adhesive coating.

According to one advantageous aspect of the disclosure, the secondary pressure sensitive adhesive pattern coating for use herein is identical to the (primary) pressure sensitive adhesive pattern coating as defined above.

According to another advantageous aspect of the disclosure, the secondary pressure sensitive adhesive pattern coating for use herein is different from the (primary) pressure sensitive adhesive pattern coating as defined above.

In one beneficial aspect of the adhesive medical article, the secondary adhesive coating for use herein forms a continuous pattern wherein the secondary adhesive uncoated areas are not interconnected. Secondary adhesive coatings forming a continuous pattern have been found to predominantly and advantageously affect the adhesive securement performance of the adhesive medical article.

In another beneficial aspect of the adhesive medical article, the secondary adhesive coating forms a discontinuous pattern wherein the secondary adhesive uncoated areas are interconnected. Secondary adhesive coatings forming a discontinuous pattern have been found to advantageously affect not only the gentleness to skin attribute but also the overall fluid management characteristics and the overall breathability of the adhesive medical article.

According to an exemplary aspect of the disclosure, the plurality of secondary adhesive uncoated areas of the central collection portion have a shape selected from the group consisting of circle, oval, square, triangle, diamond, polygon, linear stripes, non-linear stripes, curved stripes, in particular C-shape and S-shape stripes, and any combinations thereof.

According to an advantageous aspect of the disclosure, the plurality of secondary adhesive uncoated areas of the central collection portion have a shape selected from the group consisting of diamond, linear stripes, curved stripes, in particular C-shape and S-shape stripes, and any combinations thereof.

According to another exemplary aspect of the disclosure, the plurality of secondary adhesive coated areas of the central collection portion have a shape selected from the group consisting of circle, oval, square, triangle, diamond, polygon, linear stripes, non-linear stripes, curved stripes, in particular C-shape and S-shape stripes, and any combinations thereof.

According to another advantageous aspect of the disclosure, the plurality of secondary adhesive coated areas of the central collection portion have a shape selected from the group consisting of circle, oval, diamond, and any combinations thereof.

**FIG. 14** illustrates a top plan view of a square-shaped adhesive medical article **1** according to another aspect of the present disclosure, wherein the article **1** comprises a central collection portion **9** and a plurality of longitudinal extensions **10** having a non-linear shape, wherein the article is further provided with a discontinuous secondary pressure sensitive adhesive pattern coating applied onto at least part of the surface of the central collection portion **9** in such a way as to form a plurality of secondary adhesive coated areas **14** and adhesive uncoated areas **15** of the central collection portion **9,** and wherein the plurality of secondary adhesive coated areas **14** have a circle shape.

**FIG. 15** illustrates a top plan view of a square-shaped adhesive medical article **1** according to another aspect of the present disclosure, wherein the article **1** comprises a central collection portion **9** and a plurality of longitudinal extensions **10** having a non-linear shape, wherein the article is further provided with a discontinuous secondary pressure sensitive adhesive pattern coating applied onto at least part of the surface of the central collection portion **9** in such a way as to form a plurality of secondary adhesive coated areas **14** and adhesive uncoated areas **15** of the central collection portion **9,** and wherein the plurality of secondary adhesive coated areas **14** have a diamond shape.

**FIG. 16** illustrates a top plan view of a square-shaped adhesive medical article **1** according to another aspect of the present disclosure, wherein the article **1** comprises a central collection portion **9** and a plurality of longitudinal extensions **10** having a non-linear shape, wherein the article is further provided with a continuous secondary pressure sensitive adhesive pattern coating applied onto at least part of the surface of the central collection portion **9** in such a way as to form a plurality of secondary adhesive coated areas **14** and adhesive uncoated areas **15** of the central collection portion **9,** and wherein the plurality of secondary adhesive uncoated areas **15** have the shape of C-shape stripes.

**FIG. 17** illustrates a top plan view of a square-shaped adhesive medical article **1** according to another aspect of the present disclosure, wherein the article **1** comprises a central collection portion **9** and a plurality of longitudinal extensions **10** having a non-linear shape, wherein the article is further provided with a continuous secondary pressure sensitive adhesive pattern coating applied onto at least part of the surface of the central collection portion **9** in such a way as to form a plurality of secondary adhesive coated areas **14** and adhesive uncoated areas **15** of the central collection portion **9,** and wherein the plurality of secondary adhesive uncoated areas **15** have the shape of S-shape stripes.

**FIG. 18** illustrates a top plan view of a square-shaped adhesive medical article **1** according to another aspect of the present disclosure, wherein the article **1** comprises a central collection portion **9** and a plurality of longitudinal extensions **10** having a non-linear shape and provided with terminal portions **11,** wherein the article is further provided with a continuous secondary pressure sensitive adhesive pattern coating applied onto at least part of the surface of the central collection portion **9** in such a way as to form a plurality of secondary adhesive coated areas **14** and adhesive uncoated areas **15** of the central collection portion **9,** wherein the plurality of secondary adhesive uncoated areas **15** have the shape of S-shape stripes, and wherein the article **1** further comprises a single absorbent pad **12** which is connected to all the terminal portions **11.**

In one exemplary aspect, the total secondary adhesive uncoated area is no greater than 50%, no greater than 45%, no greater than 40%, no greater than 35%, no greater than 30%, no greater than 25%, no greater than 20%, no greater than 15%, no greater than 10%, or even no greater than 5%, of the total surface of the central collection portion.

In another exemplary aspect, the total secondary adhesive uncoated area is in a range from 2 to 50%, from 2 to 45%, from 5 to 45%, from 5 to 40%, from 5 to 35%, from 10 to 35%, from 10 to 30%, from 10 to 25 %, or even from 10 to 20%, of the total surface of the central collection portion.

In still another exemplary aspect, the total secondary adhesive coated area is greater than 50%, greater than 55%, greater than 60%, greater than 65%, greater than 70%, greater than 75%, greater than 80%, greater than 85%, greater than 90%, or even greater than 95%, of the total surface of the central collection portion.

In yet another exemplary aspect, the total secondary adhesive coated area is in a range from 50 to 98%, from 55 to 95%, from 55 to 90%, from 60 to 90%, from 65 to 90%, from 65 to 85%, from 70 to 85%, or even from 75 to 85%, of the total surface of the central collection portion.

According to an advantageous aspect, the adhesive medical article of the present disclosure is further provided with a support layer which substantially fully overlaps the second major surface of the backing layer.

Support layers for use herein are not particularly limited. Any support layer commonly known in the art may be used in the context of the present disclosure. Suitable support layers for use herein will be easily identified by those skilled in the art in the light of the present disclosure. Exemplary support layers for use herein comprise a material selected from the group consisting of polyethylene/vinyl acetate copolymer-coated papers, polyester films, and any combinations thereof.

Suitable support materials are preferably chosen to have advantageous rigidity, flexibility and good conformability to anatomical surfaces. Examples of support layers for use herein are described e.g. in US-A1-2001/0027285 (Heinecke et al.).

**FIG. 12** illustrates a top plan view of a square-shaped adhesive medical article **1** according to another aspect of the present disclosure, wherein the article **1** comprises a central collection portion **9** and a plurality of longitudinal extensions **10** having a non-linear shape, wherein each longitudinal extension **10** comprises at its extremity a terminal portion, wherein the article **1** is further provided with a plurality of through hole perforations **13** overlapping with a plurality of corresponding terminal portions, wherein the article **1** comprises a single absorbent pad **12** positioned on the second major surface of the backing layer and connected to all the terminal portions through all the corresponding perforations **13,** and wherein the article **1** is provided with a support layer **16** which substantially fully overlaps the second major surface of the backing layer.

**FIG. 13** is a cross-sectional view of the adhesive medical article represented in **FIG. 12** taken along the lines 13-13.

The use of a support layer which substantially fully overlaps the second major surface of the backing layer is particularly advantageous in those configurations of the adhesive medical article where the second major surface of the backing layer is provided with optional absorbent pad(s) connected to the corresponding terminal portion(s) through optional corresponding perforation(s). In those executions, the presence of a support layer overlapping and covering not only the second major surface of the backing layer but also the optional absorbent pad(s) is particularly advantageous in that it allows preserving a relatively neat external surface for the adhesive medical article.

According to a particularly advantageous aspect of the adhesive medical article, the backing layer, the primary pressure sensitive adhesive pattern coating, optionally the secondary pressure sensitive adhesive pattern coating, optionally the absorbent pad, and optionally the support layer, are translucent or transparent. Adhesive medical articles provided with these transparency characteristics advantageously improves the ability for the wound or medically treated site to remain visible not only at the time of application but also during the entire healing process.

In one advantageous aspect, the adhesive medical article of the present disclosure has an overall light-transmission (resulting from the light-transmission through the article), of at least 80%, at least 85%, or even at least 90%, when measured according to ASTM D-1003.

The adhesive article of the present disclosure may be suitably used for various medical applications. The adhesive medical article is particularly suitable for biological fluids management, in particular wound exudates. The excellent fluid management properties (e.g. control and/or transportation properties) provided by the adhesive article described herein are typically desired to maintain an aseptic environment and minimize infection risk at the wound or medically treated site, or to prevent biological fluids spillage.

The adhesive article according to the disclosure is particularly suitable for use with the so-called highly exuding wounds, such as intravenous (IV) sites or sites for gastric tubes, as well as with chronic wounds, such as e.g. diabetic foot ulcers, venous leg ulcers and pressure ulcers

In some aspects of the disclosure, the adhesive medical article is further able to provide early detection of infection due in particular to its excellent transparency characteristics.

According to an exemplary aspect of the disclosure, the adhesive medical article is selected from the group consisting of wound dressings, wound drains, intravenous access site dressings, drug delivery dressings, and any combinations thereof.

According to a preferred aspect, the adhesive medical article of the present disclosure is selected from the group of wound dressings, drug delivery dressings, and any combinations thereof.

In a particularly preferred aspect, the adhesive medical article as described herein is a wound dressing or used as a wound dressing.

According to another aspect, the present invention is directed to a method of manufacturing an adhesive medical article, comprising the steps of:
a) providing a backing layer having a first major surface and a second major surface opposite to the first major surface, and wherein the backing layer comprises a central portion and an outer circumferential portion; and
b) applying a pressure sensitive adhesive pattern coating onto at least part of the first major surface of the backing layer in such a way as to form a plurality of adhesive coated and adhesive uncoated areas of the backing layer, wherein the adhesive uncoated areas comprise a central collection portion and a plurality of longitudinal extensions radiating from the central collection portion towards the outer circumferential portion of the backing layer, wherein the pressure sensitive adhesive is selected from the group consisting of hydrophobic pressure sensitive adhesives.

In an advantageous aspect, the method as described above further comprises the steps of:
a) providing a terminal portion at the extremity of each longitudinal extension and which is positioned in the outer circumferential portion of the backing layer; and
b) providing at least one absorbent pad connected to at least one terminal portion.

In a more advantageous aspect, the method as described above further comprises the steps of:
a) providing the backing layer with at least one through hole perforation substantially overlapping with the at least one terminal portion; and
b) positioning at least one absorbent pad on the second major surface of the backing layer so as to connect the at least one absorbent pad to the at least one terminal portion through the at least one perforation.

In another more advantageous aspect, the method as described above further comprises the step of applying a secondary pressure sensitive adhesive pattern coating onto at least part of the surface of the central collection portion in such a way as to form a plurality of secondary adhesive coated and adhesive uncoated areas of the central collection portion.

According to still another aspect, the present disclosure relates to a method of applying a wound dressing to a wound, comprising the steps of:
a) providing a medical adhesive article as described above;
b) positioning the first major surface of the backing layer medical article over the wound; and
c) applying a surface pressure to the second major surface of the backing layer to conform the outer circumferential portion of the backing layer to the surface of the skin surrounding the wound.

According to yet another aspect, the present disclosure relates to a method of facilitating wound healing, comprising the steps of:
a) providing a medical adhesive article as described above;
b) positioning the first major surface of the backing layer medical article over the wound; and
c) applying a surface pressure to the second major surface of the backing layer to conform the outer circumferential portion of the backing layer to the surface of the skin surrounding the wound.

According to yet another aspect, the present disclosure is directed to a method of managing wound exudates from a wound site, comprising the steps of:
a) providing a medical adhesive article as described above;
b) positioning the first major surface of the backing layer medical article over the wound; and
c) applying a surface pressure to the second major surface of the backing layer to conform the outer circumferential portion of the backing layer to the surface of the skin surrounding the wound.

All the particular and preferred aspects relating to, in particular, the backing layer, the first major surface, the second major surface, the central portion, the (primary) pressure sensitive adhesive pattern coating, the plurality of (primary) adhesive coated and adhesive uncoated areas of the backing layer, the longitudinal extension(s), the terminal portion(s), the absorbent pad(s), the through hole perforation(s), the secondary pressure sensitive adhesive pattern coating, and the support layer, which were described hereinabove in the context of the adhesive medical article, are fully applicable to the various methods as described.

### EXAMPLES

The present disclosure is further illustrated by the following examples. These examples are merely for illustrative purposes only and are not meant to be limiting on the scope of the appended claims.

### Raw materials:

In the examples, the following raw materials and products are used:
**OHX-4070** (PDMS) is a terminal silanol functional PDMS silicone fluid (50.000 cST), commercially available from Xiameter, Midland, Michigan, USA.
**BELSIL^{®} TMS 803** is a tackifier being the co-hydrolysis product of tetraalkoxysilane (Q unit) and trimethylethoxysilane (M unit), commercially available from Wacker Chemie AG, Germany.
**TPU backing material** is a TPU-film made out of ESTANE^{®} (commercially available from Lubrizol, USA) by conventional film-coating methods, as disclosed in US-A1-2016/0015570 (Heinecke et al.).
**Stratex^{®} absorbent pad** is a rayon-based absorbent pad, commercially available from SWM, USA.

### Examples:

### General preparation of the exemplary adhesive medical articles:

The exemplary silicone-based pressure sensitive adhesive is prepared by adding the silicone oil and the tackifier in a laboratory jar and allowed to mix for at least 48 hours to produce a homogeneous composition using a PDMS/TMS 803 blending ratio of 95/5. The blended silicone-based pressure sensitive adhesive composition is then screen-printed onto the TPU backing material using a rotary screen printer (available from Stork, Charlotte, N.C.) and using the screen-printing setting and method described in U.S. Pat. No. 6,461,467 (Blatchford et al.), to obtain the targeted adhesive pattern coating. The resulting adhesive coat weight is 100 g/m² and the coating thickness is about 100 microns. After coating, the silicone-based pressure sensitive adhesive pattern is cured by e-beam radiation using suitable conditions, as described in WO 2010/056544 (Determan et al.).

### Example 1

The adhesive medical article of Example 1 is prepared according to the general procedure as described above and using a backing having a 110 mm x 110 mm dimension. The screen-printing results in the formation of a pressure sensitive adhesive pattern as depicted in **FIG. 5** and comprising twelve longitudinal extensions having a curved shape and a central collection portion having a circular shape. Each longitudinal extension has a 55 mm length of and a 2 mm width, and comprises at its extremity a terminal portion having a circular shape and a diameter of 3 mm. The central collection portion has a diameter of 8 mm.

### Example 2

The adhesive medical article of Example 2 is prepared according to the general procedure as described above and using a backing having a 110 mm x 110 mm dimension. The screen-printing results in the formation of a pressure sensitive adhesive pattern as depicted in **FIG. 9** and comprising twelve longitudinal extensions having a curved shape and a central collection portion having a circular shape. Each longitudinal extension has a 55 mm length of and a 2 mm width, and comprises at its extremity a terminal portion having a circular shape and a diameter of 3 mm. The central collection portion has a diameter of 20 mm. The adhesive medical article of Example 2 is further provided with twelve through hole perforations overlapping with the corresponding twelve terminal portions and with four rectangular-shaped absorbing pads, wherein the absorbent pads are positioned on the second major surface of the backing layer and are each connected to three terminal portions through the corresponding three perforations. The through hole perforations have a 4 mm diameter. The absorbent pads have a 60 mm x 16 mm dimension.

## Claims

1. An adhesive medical article (1) comprising a backing layer (2) having a first major surface (3) and a second major surface (4) opposite to the first major surface, wherein the backing layer comprises a central portion (5) and an outer circumferential portion (6), wherein the article further comprises a pressure sensitive adhesive pattern coating applied onto at least part of the first major surface of the backing layer in such a way as to form a plurality of adhesive coated and adhesive uncoated areas (7; 8) of the backing layer, wherein the adhesive uncoated areas (8) comprise a central collection portion (9) and at least one longitudinal extension (10) radiating from the central collection portion towards the outer circumferential portion of the backing layer, wherein the pressure sensitive adhesive is selected from the group consisting of hydrophobic pressure sensitive adhesives.

2. An article (1) according to claim 1, which comprises a plurality of longitudinal extensions (10) radiating from the central collection portion (9) towards the outer circumferential portion (6) of the backing layer (2).

3. An article (1) according to any of claim 1 or 2, wherein each longitudinal extension (10) has a curved shape, in particular the shape of a curved strip.

4. An article (1) according to any of the preceding claims, wherein the pressure sensitive adhesive is selected from the group consisting of silicone-based pressure sensitive adhesives, rubber-based pressure sensitive adhesives, acrylic-based pressure sensitive adhesives, and any combinations or mixtures thereof.

5. An article (1) according to any of the preceding claims, wherein the pressure sensitive adhesive is selected from the group consisting of silicone-based pressure sensitive adhesives, wherein the silicone base material is selected from the group of crosslinked polysiloxanes, in particular crosslinked poly diorganosiloxanes, more in particular poly dimethylsiloxanes.

6. An article (1) according to any of the preceding claims, wherein each longitudinal extension (10) comprises at its extremity a terminal portion (11) which is positioned in the outer circumferential portion (6) of the backing layer (2), and wherein the article further comprises at least one absorbent pad (12) connected to at least one terminal portion.

7. An article (1) according to claim 6, wherein the backing layer (2) is provided with at least one through hole perforation (13) overlapping with the at least one terminal portion (10), wherein the at least one absorbent pad (12) is positioned on the second major surface (4) of the backing layer (2) and is connected to the at least one terminal portion through the at least one perforation.

8. An article (1) according to any of the preceding claims, which is further provided with a secondary pressure sensitive adhesive pattern coating applied onto at least part of the surface of the central collection portion (9) in such a way as to form a plurality of secondary adhesive coated and adhesive uncoated areas (14; 15) of the central collection portion.

9. An article (1) according to claim 8, wherein the secondary adhesive coating forms a discontinuous pattern wherein the secondary adhesive uncoated areas (15) are interconnected.

10. An article (1) according to any of claim 8 or 9, wherein the plurality of secondary adhesive coated areas (14) of the central collection portion (9) have a shape selected from the group consisting of circle, oval, square, triangle, diamond, polygon, linear stripes, non-linear stripes, curved stripes, in particular C-shape and S-shape stripes, and any combinations thereof.

11. An article (1) according to any of the preceding claims, which further comprises a support layer (16) which overlaps the second major surface (4) of the backing layer (2).

12. An article (1) according to any of the preceding claims, which is selected from the group consisting of wound dressings, wound drains, intravenous access site dressings, drug delivery dressings, and any combinations thereof.

13. A method of manufacturing a medical adhesive article (1), comprising the steps of:
a) providing a backing layer (2) having a first major surface (3) and a second major surface (4) opposite to the first major surface, and wherein the backing layer comprises a central portion (5) and an outer circumferential portion (6); and
b) applying a pressure sensitive adhesive pattern coating onto at least part of the first major surface of the backing layer in such a way as to form a plurality of adhesive coated and adhesive uncoated areas (7; 8) of the backing layer, wherein the uncoated areas (8) comprise a central collection portion (9) and a plurality of longitudinal extensions (10) radiating from the central collection portion towards the outer circumferential (peripheral) portion of the backing layer,
wherein the pressure sensitive adhesive is selected from the group consisting of hydrophobic pressure sensitive adhesives.

14. A method according to claim 13, which further comprises the steps of:
a) providing a terminal portion (11) at the extremity of each longitudinal extension (10) and which is positioned in the outer circumferential portion (6) of the backing layer (2); and
b) providing at least one absorbent pad (12) connected to at least one terminal portion.

## Patentansprüche

1. Ein medizinischer Klebergegenstand (1), aufweisend eine Trägerschicht (2), die eine erste Hauptoberfläche (3) und eine zweite Hauptoberfläche (4) gegenüber der ersten Hauptoberfläche hat, wobei die Trägerschicht einen Mittelteil (5) und einen Außenumfangsabschnitt (6) aufweist, wobei der Gegenstand ferner eine Haftklebermusterbeschichtung aufweist, die auf mindestens einen Teil der ersten Hauptoberfläche der Trägerschicht derart aufgebracht ist, dass eine Mehrzahl von kleberbeschichteten und kleberunbeschichteten Bereichen (7; 8) der Trägerschicht gebildet wird, wobei die kleberunbeschichteten Bereiche (8) einen Mittelsammelteil (9) und mindestens eine Längsverlängerung (10) aufweisen, die von dem Mittelsammelteil zu dem Außenumfangsabschnitt der Trägerschicht hin abstrahlen, wobei der Haftkleber aus der Gruppe ausgewählt ist, bestehend aus hydrophoben Haftklebern.

2. Ein Gegenstand (1) nach Anspruch 1, der eine Mehrzahl von Längsverlängerungen (10) aufweist, die von dem Mittelsammelteil (9) zu dem Außenumfangsabschnitt (6) der Trägerschicht (2) hin abstrahlt.

3. Ein Gegenstand (1) nach einem der Ansprüche 1 oder 2, wobei jede Längsverlängerung (10) eine gekrümmte Form hat, insbesondere die Form eines gekrümmten Streifens.

4. Ein Gegenstand (1) nach einem der vorstehenden Ansprüche, wobei der Haftkleber aus der Gruppe ausgewählt ist, bestehend aus silikonbasierten Haftklebern, kautschukbasierten Haftklebern, acrylbasierten Haftklebern und beliebigen Kombinationen davon.

5. Ein Gegenstand (1) nach einem der vorstehenden Ansprüche, wobei der Haftkleber aus der Gruppe ausgewählt ist, bestehend aus silikonbasierten Haftklebern, wobei das Silikonbasismaterial aus der Gruppe von vernetzten Polysiloxanen, insbesondere vernetzten Polydiorganosiloxanen, insbesondere Polydimethylsiloxanen, ausgewählt ist.

6. Ein Gegenstand (1) nach einem der vorstehenden Ansprüche, wobei jede Längsverlängerung (10) an ihrem äußersten Ende einen Endabschnitt (11) aufweist, der in dem Außenumfangsabschnitt (6) der Trägerschicht (2) positioniert ist, und wobei der Gegenstand ferner mindestens ein Absorptionskissen (12) aufweist, das mit mindestens einem Endabschnitt verbunden ist.

7. Ein Gegenstand (1) nach Anspruch 6, wobei die Trägerschicht (2) mit mindestens einer Durchgangsperforation (13) versehen ist, die sich mit dem mindestens einen Endabschnitt (10) überlappt, wobei das mindestens eine Absorptionskissen (12) auf der zweiten Hauptoberfläche (4) der Trägerschicht (2) positioniert ist und mit dem mindestens einen Endabschnitt durch die mindestens eine Perforation verbunden ist.

8. Ein Gegenstand (1) nach einem der vorstehenden Ansprüche, der ferner mit einer sekundären Haftklebermusterbeschichtung versehen ist, die auf mindestens einen Teil der Oberfläche des Mittelsammelteils (9) derart aufgebracht ist, dass eine Mehrzahl von sekundären kleberbeschichteten und kleberunbeschichteten Bereichen (14; 15) des Mittelsammelteils gebildet wird.

9. Ein Gegenstand (1) nach Anspruch 8, wobei die sekundäre Kleberbeschichtung ein diskontinuierliches Muster bildet, wobei die sekundären kleberunbeschichteten Bereiche (15) miteinander verbunden sind.

10. Ein Gegenstand (1) nach einem der Ansprüche 8 oder 9, wobei die Mehrzahl von sekundären kleberbeschichteten Bereiche (14) des Mittelsammelteils (9) eine Form hat, die aus der Gruppe ausgewählt ist, bestehend aus Kreis, Oval, Quadrat, Dreieck, Raute, Polygon, linearen Streifen, nichtlinearen Streifen, gekrümmten Streifen, insbesondere C-förmigen und S-förmigen Streifen, und beliebigen Kombinationen davon.

11. Ein Gegenstand (1) nach einem der vorstehenden Ansprüche, der ferner eine Stützschicht (16) aufweist, die die zweite Hauptoberfläche (4) der Trägerschicht (2) überlappt.

12. Ein Gegenstand (1) nach einem der vorstehenden Ansprüche, der aus der Gruppe ausgewählt ist, bestehend aus Wundverbänden, Wunddrainagen, Verbänden für intravenöse Zugangsstellen, Verbänden für eine Arzneimittelabgabe und beliebigen Kombinationen davon.

13. Ein Verfahren zum Herstellen eines medizinischen Klebergegenstands (1), aufweisend die Schritte:
a) Bereitstellen einer Trägerschicht (2), die eine erste Hauptoberfläche (3) und eine zweite Hauptoberfläche (4) gegenüber der ersten Hauptoberfläche hat, und wobei die Trägerschicht einen Mittelteil (5) und einen Außenumfangsabschnitt (6) aufweist; und
b) Aufbringen einer Haftklebermusterbeschichtung auf mindestens einen Teil der ersten Hauptoberfläche der Trägerschicht derart, dass eine Mehrzahl von kleberbeschichteten und kleberunbeschichteten Bereichen (7; 8) der Trägerschicht gebildet wird, wobei die unbeschichteten Bereiche (8) einen Mittelsammelteil (9) und eine Mehrzahl von Längsverlängerungen (10) aufweisen, die von dem Mittelsammelteil zu dem Außenumfangs(peripherie)abschnitt der Trägerschicht hin abstrahlen,
wobei der Haftkleber aus der Gruppe ausgewählt ist, bestehend aus hydrophoben Haftklebern.

14. Ein Verfahren nach Anspruch 13, das ferner die Schritte aufweist:
a) Bereitstellen eines Endabschnitts (11) an dem äußersten Ende jeder Längsverlängerung (10) und der in dem Außenumfangsabschnitt (6) der Trägerschicht (2) positioniert ist; und
b) Bereitstellen mindestens eines Absorptionskissens (12), das mit mindestens einem Endabschnitt verbunden ist.

## Revendications

1. Article médical adhésif (1) comprenant une couche de soutien (2) ayant une première surface principale (3) et une seconde surface principale (4) à l'opposé de la première surface principale, dans lequel la couche de soutien comprend une partie centrale (5) et une partie circonférentielle externe (6), l'article comprenant en outre un revêtement de motif d'adhésif sensible à la pression appliqué sur au moins une partie de la première surface principale de la couche de soutien d'une manière telle que sont formées une pluralité de zones revêtues d'adhésif et non revêtues d'adhésif (7; 8) de la couche de soutien, dans lequel les zones non revêtues d'adhésif (8) comprennent une partie de collecte centrale (9) et au moins une extension longitudinale (10) rayonnant à partir de la partie de collecte centrale vers la partie circonférentielle externe de la couche de soutien, dans lequel l'adhésif sensible à la pression est choisi dans le groupe constitué d'adhésifs sensibles à la pression hydrophobes.

2. Article (1) selon la revendication 1, qui comprend une pluralité d'extensions longitudinales (10) rayonnant à partir de la partie de collecte centrale (9) vers la partie circonférentielle externe (6) de la couche de soutien (2).

3. Article (1) selon l'une quelconque de la revendication 1 ou 2, dans lequel chaque extension longitudinale (10) a une forme courbe, en particulier la forme d'une bande courbe.

4. Article (1) selon l'une quelconque des revendications précédentes, dans lequel l'adhésif sensible à la pression est choisi dans le groupe constitué d'adhésifs sensibles à la pression à base de silicone, adhésifs sensibles à la pression à base de caoutchouc, adhésifs sensibles à la pression à base d'acrylique, et l'un quelconque de combinaisons ou mélanges de ceux-ci.

5. Article (1) selon l'une quelconque des revendications précédentes, dans lequel l'adhésif sensible à la pression est choisi dans le groupe constitué d'adhésifs sensibles à la pression à base de silicone, le matériau de base silicone étant choisi dans le groupe de polysiloxanes réticulés, en particulier polydiorganosiloxanes réticulés, plus particulièrement polydiméthylsiloxanes.

6. Article (1) selon l'une quelconque des revendications précédentes, dans lequel chaque extension longitudinale (10) comprend au niveau de son extrémité une partie terminale (11) qui est positionnée dans la partie circonférentielle externe (6) de la couche de soutien (2), et l'article comprenant en outre au moins un tampon absorbant (12) relié à au moins une partie terminale.

7. Article (1) selon la revendication 6, dans lequel il est fourni à la couche de soutien (2) au moins une perforation de trou passant (13) chevauchant l'au moins une partie terminale (10), dans lequel l'au moins un tampon absorbant (12) est positionné sur la seconde surface principale (4) de la couche de soutien (2) et est relié à l'au moins une partie terminale en passant par l'au moins une perforation.

8. Article (1) selon l'une quelconque des revendications précédentes, auquel est en outre fourni un revêtement de motif d'adhésif sensible à la pression secondaire appliqué sur au moins une partie de la surface de la partie de collecte centrale (9) d'une manière telle que sont formées une pluralité de zones revêtues d'adhésif et non revêtues d'adhésif secondaires (14; 15) de la partie de collecte centrale.

9. Article (1) selon la revendication 8, dans lequel le revêtement d'adhésif secondaire forme un motif discontinue dans lequel les zones non revêtues d'adhésif secondaires (15) sont reliées entre elles.

10. Article (1) selon l'une quelconque de la revendication 8 ou 9, dans lequel la pluralité de zones revêtues d'adhésif secondaires (14) de la partie de collecte centrale (9) ont une forme choisie dans le groupe constitué de cercle, ovale, carré, triangle, diamant, polygone, bandes linéaires, bandes non linéaires, bandes courbes, en particulier bandes à forme en C et à forme en S, et l'une quelconque des combinaisons de ceux-ci.

11. Article (1) selon l'une quelconque des revendications précédentes, qui comprend en outre une couche de support (16) qui chevauche la seconde surface principale (4) de la couche de soutien (2).

12. Article (1) selon l'une quelconque des revendications précédentes, qui est choisi dans le groupe constitué de pansements pour plaie, drains de plaie, pansements de site d'accès intraveineux, pansements d'administration de médicament, et l'une quelconque des combinaisons de ceux-ci.

13. Procédé de fabrication d'un article adhésif médical (1), comprenant les étapes consistant à:
a) fournir une couche de soutien (2) ayant une première surface principale (3) et une seconde surface principale (4) à l'opposé de la première surface principale, et la couche de soutien comprenant une partie centrale (5) et une partie circonférentielle externe (6); et
b) appliquer un revêtement de motif d'adhésif sensible à la pression sur au moins une partie de la première surface principale de la couche de soutien d'une manière telle que sont formées une pluralité de zones revêtues d'adhésif et non revêtues d'adhésif (7; 8) de la couche de soutien, les zones non revêtues (8) comprenant une partie de collecte centrale (9) et une pluralité d'extensions longitudinales (10) rayonnant à partir de la partie de collecte centrale vers la partie circonférentielle externe (périphérique) de la couche de soutien,
dans lequel l'adhésif sensible à la pression est choisi dans le groupe constitué d'adhésifs sensibles à la pression hydrophobes.

14. Procédé selon la revendication 13, qui comprend en outre les étapes consistant à:
a) fournir une partie terminale (11) au niveau de l'extrémité de chaque extension longitudinale (10) et qui est positionnée dans la partie circonférentielle externe (6) de la couche de soutien (2); et
b) fournir au moins un tampon absorbant (12) relié à au moins une partie terminale.
